(19) Europäisches Patentamt
European Patent Office
Office européen des brevets

(11) **EP 2 170 098 B1**

(12) **EUROPÄISCHE PATENTSCHRIFT**

(45) Veröffentlichungstag und Bekanntmachung des Hinweises auf die Patenterteilung:
**15.07.2015  Patentblatt 2015/29**

(21) Anmeldenummer: **08784924.6**

(22) Anmeldetag: **21.07.2008**

(51) Int Cl.:
*A23K 1/16* *(2006.01)*          *A61K 31/195* *(2006.01)*
*A23K 1/00* *(2006.01)*

(86) Internationale Anmeldenummer:
**PCT/EP2008/005958**

(87) Internationale Veröffentlichungsnummer:
**WO 2009/012960 (29.01.2009 Gazette 2009/05)**

(54) **ABRIEBFESTE UND RIESELFÄHIGE GLYCOCYAMIN-HALTIGE FORMLINGE UND VERFAHREN ZU DEREN HERSTELLUNG**

ABRASION-RESISTANT FREE-FLOWING GLYCOCYAMINE-CONTAINING MOULDINGS AND METHODS FOR THEIR PRODUCTION

PRODUITS MOULÉS CONTENANT DE LA GLYCOCYAMINE, RÉSISTANTS À L'USURE PAR FROTTEMENT ET APTES À L'ÉCOULEMENT, ET PROCÉDÉ DE PRODUCTION

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(30) Priorität: **21.07.2007  DE 102007034102**

(43) Veröffentlichungstag der Anmeldung:
**07.04.2010  Patentblatt 2010/14**

(73) Patentinhaber: **AlzChem AG**
**83308 Trostberg (DE)**

(72) Erfinder:
• **WINKLER, Stephan**
  **83352 Altenmarkt (DE)**
• **MÖLLER, Roland**
  **83376 Truchtlaching (DE)**
• **ERL, Susanne**
  **83342 Peterskirchen (DE)**

(74) Vertreter: **Weickmann & Weickmann**
**Postfach 860 820**
**81635 München (DE)**

(56) Entgegenhaltungen:
**WO-A-2005/120246     WO-A-2006/092298**

EP 2 170 098 B1

**Beschreibung**

Die vorliegende Erfindung betrifft rieselfähige und abriebfeste Glycocyamin-haltige Formlinge sowie Verfahren zu deren Herstellung.

Glycocyamin ist eine in Wirbeltieren und auch im Menschen vorkommende körpereigene Substanz, welche bei der Biosynthese des Kreatins eine zentrale Rolle einnimmt. Kreatin kann sowohl durch die Nahrung aufgenommen als auch endogen gebildet werden, wobei es als energiereiches Phosphokreatin neben dem Adenosintriphosphat (ATP) eine wesentliche Energiereserve des Muskels darstellt. Im Ruhezustand des Muskels kann ATP auf Kreatin eine Phosphat-Gruppe übertragen, wobei Phosphokreatin gebildet wird, welches dann im direkten Gleichgewicht mit ATP steht. Bei Muskelarbeit ist es von entscheidender Bedeutung, die ATP-Vorräte schnellstmöglich wieder aufzufüllen. Hierfür steht in den ersten Sekunden maximaler Muskelbelastung das Phosphokreatin zur Verfügung. Dieses kann in einer sehr schnellen Reaktion, durch das Enzym Kreatinkinase, eine Phosphatgruppe auf Adenosindiphosphat übertragen und somit ATP zurückbilden. Dies wird auch als Lohmann-Reaktion bezeichnet.

Positive Effekte einer Supplementierung mit Kreatin Monohydrat sind beim Menschen seit vielen Jahren vor allem im Bereich der Sporternährung, aber auch im medizinischen Bereich bekannt. Positive Effekte einer Nahrungsergänzung wurden auch bei Tieren gefunden und Kreatin-Monohydrat wurde deshalb für die Verwendung als Futtermittelzusatz und als Fleischmehlersatz in der Tierernährung empfohlen. Seit dem Verbot von tierischen Eiweißen in Futtermitteln ab dem Jahre 2000 in der EU wurden viele Diäten für Zucht- und Masttiere auf rein vegetarische Diäten umgestellt, wobei auch weitgehend auf Fischmehl verzichtet wurde, welches in dem Verbot nicht eingeschlossen ist. Die Umstellung auf rein vegetarische Diäten führte zu Einbußen in der Leistung und auch nach sieben Jahren sind die rein vegetarischen Diäten denen mit tierischen Eiweißen unterlegen. Ein Grund dieser Unterlegenheit ist der Mangel an Kreatin. Frühere Versuche konnten klar zeigen, dass dem Futter zugesetztes Kreatin-Monohydrat die Mastleistung verbessern kann, wenn rein vegetarische Diäten verfüttert werden [Wallimann, T.; Pfirter, H.P.: Use of Creatine as a Feed Additive. EP1051914].

Neben den unzweifelhaft positiven Effekten besitzt Kreatin-Monohydrat auch einige Nachteile. Die Stabilität dieser Verbindung in wässrigen Lösungen ist sehr begrenzt und Kreatin-Monohydrat besitzt nach oraler Aufnahme nur eine geringe Bioverfügbarkeit. Weiterhin ist Kreatin-Monohydrat eine sehr teure Substanz und die erzielten Leistungsverbesserungen im Tiermastbereich werden nahezu durch die Kosten kompensiert. In jüngster Zeit wurde deshalb auch Glycocyamin als Nahrungsergänzungsmittel und Futtermittel eingesetzt, welches im Vergleich zum Kreatin in wässriger Lösung eine erstaunliche Stabilität besitzt und wesentlich besser bioverfügbar ist [Gastner, T.; Krimmer, H.-P.: Guanidinoacetic acid used as an animal food additive. EP1758463]. Glycocyamin wird im Körper sehr effizient und schnell in Kreatin umgewandelt. Es kann deshalb bei gleicher Wirkung in wesentlich geringeren Mengen als Kreatin verabreicht werden.

In der Literatur wurden bereits eine Vielzahl von Syntheseverfahren zur Herstellung von Glycocyamin beschrieben. Bereits 1861 wurde von Strecker die Synthese von Glycocyamin aus Glycin und Cyanamid erstmals erfolgreich durchgeführt [Strecker, M.; Jahresber. Fortschr. Chem. Verw., (1861), 530].

In Analogie beschrieb Fromm 1925 die Umsetzung von Glycinhydrochlorid mit Natriumcyanamid und Salzsäure zu Glycocyamin-Hydrochlorid [Fromm, E.; Justus Liebigs Ann. Chem., 442, (1925), 130-149].

Cyanamid wurde auch von Vassel in zwei Patenten zur Herstellung von Glycocyamin verwendet. Es wurde mit Glycin umgesetzt, wobei der pH mit Natriumhydroxid auf 9,4 eingestellt wurde [Vassel, B.; US2654779]. Weiterhin schlug Vassel die Verwendung von Chloressigsäure und Ammoniak vor. Hierbei wurde zunächst Glycinhydrochlorid gebildet. Die so erhaltene Lösung wurde anschließend mit Natronlauge auf einen pH > 9 eingestellt und nachfolgend mit Cyanamid umgesetzt [Vassel, B.; US2620354].

1903 wurde die Herstellung von Glycocyamin von Wheeler und Merriam durch die Umsetzung von Glycin mit S-Methylisothioharnstoffiodid in basischer, wässriger Lösung beschrieben. Hierbei wurde Kaliumhydroxid als Base eingesetzt [Wheeler, H.I.; Merriam, H.F.; Am. Chem. Journal, 29, (1903), 478-492.]. Ein sehr ähnliches Vorgehen beschrieb Fischl 1934, wobei als Base ein Überschuss an Ammoniak verwendet wurde [Fischl, S.; (1931), US1967400].

Bei den bekannten Verfahren fällt Glycocyamin in der Regel als feinkristallines weißes bis leicht gelbliches Pulver an, welches einen erheblichen Staubanteil (Partikel < 63 $\mu$m) besitzt. Der mittlere Korndurchmesser kann in weiten Bereichen schwanken und liegt in der Regel zwischen 25 und 150 $\mu$m. Das aus den bekannten Synthesen erhaltene Glycocyamin hat einen Staubanteil von über 50 Gew.-% und eine Rieselfähigkeit der Note 6 und kann deshalb für die industrielle Herstellung von Futtermitteln praktisch nicht eingesetzt werden.

Die Rieselfähigkeit von Pulvern, Granulaten und Extrudaten kann beispielsweise über das Fließverhalten durch Prüftrichter mit verschiedenen Auslassdurchmessern bestimmt werden (Feed Tech 9.10/2005, Seite 23-26; siehe auch Beispielteil der vorliegenden Erfindung). Die Rieselfähigkeit wird hierbei mit Noten von 1 für sehr gutes Fließverhalten bis 6 für sehr schlechtes Fließverhalten beurteilt. Für die Herstellung von industriellen Futtermitteln sollten die eingesetzten Feststoffe mindestens die Note 3 erreichen.

Für die Abriebfestigkeit von Futtermittel-Granulaten und Extrudaten wurden bisher keine einheitlichen Prüfprotokolle festgelegt. Eine Methode, welche sich in der Praxis als reproduzierbar und aussagekräftig erwiesen hat, ist der Abrieb (Partikel < 63 $\mu$m) in einem Luftstrahlsieb über eine definierte Zeit mit einem definierten Unterdruck. Insbesondere hat sich die Messung der Differenz des Abriebs nach 3 und 15 Minuten bei einem Unterdruck von 7200 Pascal etabliert. Bei guten Granulaten liegt dieser Wert bei unter 5 Gew.-%. Die Methode wird im Beispielteil näher erläutert.

Aufgabe der vorliegenden Erfindung war deshalb die Bereitstellung rieselfähiger, abriebfester und somit staubarmer Produkte, welche sich insbesondere für die Einarbeitung in Futtermittel eignen, sowie Verfahren zu deren Herstellung.

Diese Aufgabe wurde erfindungsgemäß durch Bereitstellung von rieselfähigen und abriebfesten Glycocyaminhaltigen Formlingen mit einem Schüttgewicht zwischen 350 und 850 kg/m$^3$, einem Kornspektrum von 32 bis 2750 $\mu$m und einem Glycocyamingehalt von 55 bis 99,9 Gew.-%, bezogen auf das Gesamtgewicht gelöst.

Es konnte gezeigt werden, dass mit der Bereitstellung der erfindungsgemäßen Glycocyamin-haltigen Formlinge die Aufgabenstellung voll erfüllt werden konnte, nämlich rieselfähige, abriebfeste und somit staubarme Produkte für die Futtermittelindustrie bereitzustellen, welche sich durch eine gute Handhabbarkeit auszeichnen.

Gemäß einer bevorzugten Ausführungsform handelt es sich um Granulate und Extrudate mit einem Schüttgewicht zwischen 400 und 800 kg/m$^3$ und insbesondere zwischen 450 und 750 kg/m$^3$. Weiterhin ist als bevorzugt anzusehen, dass das Kornspektrum der beanspruchten Formlinge zwischen 32 und 1000 $\mu$m liegt und vorzugsweise weniger als 10 Gew.-% der Partikel unter 100 $\mu$m und weniger als 10 Gew.-% der Partikel über 850 $\mu$m liegen. Die Formlinge haben vorzugsweise einem Glycocyamingehalt von 85 bis 99 Gew.-%, insbesondere 95 bis 98,5 Gew.-%.

In einer bevorzugten Ausführungsform enthalten die Formlinge organische oder anorganische Bindemittel in Mengen von 0,05 bis 15 Gew.-%, insbesondere 0,1 bis 1,5 Gew.-%, welche sich für den Einsatz der erfindungsgemäßen Produkte als Futtermitteladditive eignen. Vorzugsweise handelt es sich bei den für die Herstellung von Formlingen verwendeten Bindemitteln um Nebenprodukte oder Ausgangssubstanzen aus dem Herstellprozess des Glycocyamins, wie etwa Glycin oder Salze des Glycocyamins, so dass diese zur Reinigung des Produktes vorher nicht abgetrennt werden müssen. Als besonders vorteilhaft hat es sich erwiesen, wenn dem eingesetzten Glycocyamin noch geringe Mengen dieser Stoffe in gelöster Form anhaften, wobei es sich auch um in Wasser gelöstes Glycocyamin selbst handeln kann.

Insbesondere geeignet ist auch die Zugabe weiterer Bindemittel, wie etwa Methylcellulose, Ethylcellulose, Carboxymethylcellulose, Carboxyethylcellulose, Carboxypropylcellulose, Hydroxypropylmethylcellulose, Hydroxymethylcellulose, mikrokristalliner Cellulose, Ethylmethylcellulose und andere Cellulose-Derivate, Stärke, Hydroxypropylstärke, nativer Stärke, pregelatinisierter oder modifizierter Stärke, Zucker, Zuckersirup, Dextrin, Gelatine, Propylvinylalkohol, Polyvinylpyrrolidon, Xanthan, Glycocyamin-Salze Gummiarabicum, Natriumchlorid, Natriumcarbonat, Natriumhydrogencarbonat und Glycerin sowie deren Mischungen.

Zur Verbesserung der Fließfähigkeit der Formlinge kann es von Vorteil sein, dass diese ein Fließhilfsmittel, insbesondere eine hydrophile und/oder hydrophobe Kieselsäure und/oder Zuschlagsstoffe auf silikatischer Basis und/oder Fettsäuren und/oder deren Salze, wie Stearinsäure oder Palmitinsäure sowie deren Natrium-, Kalium- und Calcium-Salze enthalten. Die Fließhilfsmittel und Bindemittel werden dem Glycocyamin in trockener Form, als Suspension oder Lösung vor der Formgebung zugesetzt, wobei sich Mengen von 0,01 bis 5 Gew.-% als geeignet erwiesen haben.

Die vorliegende Erfindung sieht weiterhin vor, dass die Glycocyamin-haltigen Formlinge optional bis zu 40 Gew.-%, insbesondere 1 bis 10 Gew.-% eines weiteren ernährungsphysiologisch wirksamen Stoffes der Reihe Kohlenhydrate, Fette, Aminosäuren, Proteine, Vitamine, Mineralstoffe, Spurenelemente sowie deren Derivate und Mischungen daraus enthalten können. Als bevorzugt sind insbesondere die essentiellen Aminosäuren Lysin, Threonin, Methionin und Tryptophan anzusehen, weiterhin Vitamin A, Vitamin D3, Vitamin E, Nicotinsäure, Nicotinsäureamid, $\beta$-Carotin, Fischmehl und Kasein.

Die erfindungsgemäßen Glycocyamin-haltigen Formlinge sollten vorzugsweise eine Rieselfähigkeit der Note 3, besonders bevorzugt der Note 2 oder 1 besitzen sowie eine Abriebfestigkeit von unter 12 Gew.-%, vorzugsweise unter 10 Gew.-% und besonders bevorzugt unter 4 Gew.-%.

Ein weiterer Gegenstand der Erfindung ist ein Verfahren zur Herstellung von Glycocyamin-haltigen Formlingen, dadurch gekennzeichnet, dass diese durch Verformung, insbesondere Mischgranulation oder formgebende Extrudierung einer Zusammensetzung aus Glycocyamin und Wasser und anschließendes Trocknen erhalten werden. Die erfindungsgemäßen Verfahren können sowohl kontinuierlich als auch als Batch-Verfahren durchgeführt werden.

Als besonders geeignet für die Verformung haben sich Extruder, insbesondere Einschneckenextruder, Zweischneckenextruder, Ringmatrizenpressen und Kollerwerke erwiesen. Der eingesetzte Feststoff wird hierbei im Allgemeinen bei Drücken bis 80 bar und Temperaturen von 20 bis 120 °C durch eine Extrusionsmatrize gepresst. Die Extrudatgröße kann entweder durch mechanisches Abschneiden eingestellt werden oder es tritt ein Zerfall der Extrudate bei geeigneter Wahl der Verfahrensparameter auf. Hierdurch können Extrudate zwischen 32 und 2750 $\mu$m, insbesondere zwischen 32 und 1000 $\mu$m erzeugt werden.

Als besonders geeignet für die Verformung haben sich weiterhin Granulatoren, insbesondere Intensivmischer, Vertikal-Granulatoren, Sprühgranulatoren, Ringschichtgranulatoren und Pflugscharmischer erwiesen. Der eingesetzte Feststoff wird hierbei hohen Scherkräften ausgesetzt, wobei sich je nach Art, Größe und Kapazität des Granulators Geschwindigkeiten von 300 bis 2500 Umdrehungen pro Minute als geeignet erwiesen haben. Die Granulierung kann bei Temperaturen zwischen 20 und 120°C durchgeführt werden, wobei das beschriebene Verfahren Granulate zwischen 32 und 2750 μm, insbesondere zwischen 32 und 1000 μm liefert.

Vorteilhaft wird das zur Herstellung der Formlinge verwendete Glycocyamin aus Glycin und Cyanamid in einem wässrigen Lösungsmittel, insbesondere Wasser, unter Zugabe einer Base hergestellt. Verfahren dieses Typs werden beispielsweise in US 2,654,779 und US 2,620,354 beschrieben. In einer bevorzugten Ausführungsform wird für die Granulation und Extrusion ein wasserfeuchtes Glycocyamin direkt aus dem Herstellprozess eingesetzt, an welchem noch gelöstes Glycocyamin und/oder Ausgangs- oder Nebenprodukte aus dem Herstellprozess anhaften. Eine Restfeuchte von 15 bis 25 Gew.-% des eingesetzten Materials hat sich hierbei als besonders vorteilhaft erwiesen. Insgesamt können die für die Formgebung eingesetzten Mischungen zwischen 40 und 93 Gew.-% Glycocyamin, zwischen 7 und 60 Gew.-% Wasser, zwischen 0 und 15 Gew.-% Bindemittel sowie 0 bis 40 Gew.-% eines weiteren ernährungsphysiologisch wirksamen Stoffes enthalten.

Es hat sich sowohl für die Granulation als auch für die Extrusion als besonders vorteilhaft erwiesen, wenn das verwendete Glycocyamin einen mittleren Korndurchmesser von < 95 μm, vorzugsweise < 25 μm und insbesondere < 15 μm besitzt. Weiterhin erwies sich vor allem amorphes Glycocyamin als besonders geeignet. Für die Erfindung ist es deshalb als bevorzugt anzusehen, dass mehr als 40%, bevorzugt mehr als 90% des eingesetzten Materials in amorpher Form vorliegt. Besonders geeignetes amorphes Glycocyamin für die Granulierung lässt sich zum einen durch Einstellung geeigneter Prozessparameter im Herstellprozess, welche dem Fachmann bekannt sind, als auch durch Vermahlen von kristallinem Material erzeugen.

Um ein stabiles, trockenes Granulat bzw. Extrudat zu erhalten, werden die Formlinge getrocknet. Hierbei haben sich vor allem Fließbetttrockner und Wirbelschichttrockner als besonders schonend erwiesen, um eine mechanische Zerstörung der noch feuchten Formlinge zu vermeiden. Vorzugsweise werden Temperaturen zwischen 50 und 130°C und ggf. Vakuum eingesetzt.

Der Staubehalt der erhaltenen Granulate und Extrudate liegt unter 5 Gew.-%, vorzugsweise unter 2 Gew.-%, gemessen nach der Methode von Dr. Groschopp.

Aufgrund der sehr guten Abriebfestigkeit und Rieselfähigkeit eigenen sich die erfindungsgemäßen Glycocyamin-haltigen Formlinge hervorragend als Futtermitteladditiv.

Die nachfolgenden Beispiele sollen die vorliegende Erfindung näher veranschaulichen.

Beispiele

1. Methoden zur Bestimmung von Abriebfestigkeit und Rieselfähigkeit

1.1 Abriebfestigkeit

Die Prüfmittel bestehen aus

- Luftstrahlsieb
- Analysensieb 63 μm
- Analysenwaage (Genauigkeit 0,01 g)
- Industriestaubsauger
- Wägeschale

25 g vom zu bestimmenden Granulat oder Extrudat werden eingewogen und für 3 min bei 7200 Pascal Unterdruck auf dem Luftstrahlsieb gesiebt und im Anschluss wieder zurückgewogen. Die Differenz (= AW1) ist gleich die Abriebfestigkeit nach 3 min. Anschließend wird dieser Vorgang mit derselben Probe und bei denselben Einstellungen für 12 min Siebzeit wiederholt und die Probe zurückgewogen. Die Differenz zur Einwaage (= AW2) ist gleich die Abriebfestigkeit nach 15 min.

$$R_x = \frac{(EW - AW_x) * 100}{EW}$$

R  = Abriebfestigkeit [%]
AW  = Auswaage nach Absiebung [g]
EW  = Einwaage [g]

Die Differenz aus den Werten bei 3 min und 15 min ist ein Maß für die Abriebfestigkeit. Je höher dieser Wert, desto mehr Abrieb wird erzeugt.

1.2 Rieselfähigkeit

Das Prüfmittel besteht aus fünf Prüftrichtern mit gleichem Durchmesser und Neigungswinkel, jedoch mit verschiedenen Auslassdurchmessern (2,5 mm; 5 mm; 8 mm; 12 mm und 18 mm). Der zu bestimmende Feststoff wird dazu in die Prüftrichter gefüllt, wobei der Auslass von unten verschlossen wird, damit beim Befüllen kein Gut auslaufen kann. Im nächsten Schritt wird der Auslass - ohne den Prüftrichter zu erschüttern - vollständig geöffnet, so dass der komplette Auslassquerschnitt freigegeben wird. Bewertungsgröße ist der Durchmesser, bei dem der Feststoff selbständig und ohne Fremdeinwirkung durchrieselt. Dabei gilt:

- Feststoff rieselt durch den 2,5 mm Auslass: Note 1

- Feststoff rieselt durch den 5 mm Auslass: Note 2

- Feststoff rieselt durch den 8 mm Auslass: Note 3

- Feststoff rieselt durch den 12 mm Auslass: Note 4

- Feststoff rieselt durch den 18 mm Auslass: Note 5

- Feststoff rieselt nicht durch den 18 mm Auslass: Note 6

2. Herstellung von Granulaten und Extrudaten

2.1 In einem 75 Liter Intensivmischer (Fa. Eirich) wurden 34 kg Glycocyamin (KGA x50-Wert (mittlerer Korndurchmesser) = 13,6 $\mu$m) mit einem Wassergehalt von 20,8 % bei Raumtemperatur vorgelegt und 1 min homogenisiert. Anschließend wurde unter langsamem Rühren 269 g Stärke zugegeben. Danach wurde der Mischerinhalt bei 1500 Upm gerührt, dabei fand eine Temperaturerhöhung auf ca. 50°C statt. Nach 5 min Granulierzeit wurde ein Granulat im gewünschten Korngrößenbereich erhalten. Das erhaltene Glycocyamin-Granulat wurde im Wirbelschichttrockner bis zu einer Produkttemperatur von 80 °C getrocknet und im Anschluss der Grobanteil über 1,00 mm abgesiebt.

2.2 In einem Vertikal-Granulator wurden 35 kg Glycocyamin (KGA x50-Wert = 23,2 $\mu$m) mit einer Restfeuchte von 13 % bei Raumtemperatur vorgelegt und 1 min homogenisiert. Anschließend wurde unter langsamem Rühren 305 g Stärke und 2,60 kg Wasser zugegeben. Danach wurde der Mischerinhalt bei 2000 Upm gerührt, dabei fand eine Temperaturerhöhung statt. Nach 8 min Granulierzeit wurde ein Granulat im gewünschten Korngrößenbereich erhalten. Das Granulat wurde im Vakuumtrockenschrank bei 80 °C und 50 mbar getrocknet.

2.3 Es wurden 4,3 kg Glycocyamin (KGA x50-Wert = 12,6 $\mu$m) mit einer Restfeuchte von 20,7 % in einem Mischer vorgelegt und unter langsamen Rühren 34 g Stärke eingemischt. Anschließend wurde die Mischung in einen Ringmatrizenextruder gegeben und durch eine Matrize mit 0,7 mm Bohrungen gepresst. Das dadurch erhaltene Extrudat wurde auf einem Wirbelschichttrockner bis zu einer Produkttemperatur von 50 °C getrocknet.

2.4 In einem 75 Liter Intensivmischer (Fa. Eirich) wurden 34 kg Glycocyamin (KGA x50-Wert = 63,8 $\mu$m) mit einer Restfeuchte von 9,4 % bei Raumtemperatur vorgelegt und 1 min homogenisiert. Anschließend wurde unter langsamem Rühren 308 g Stärke und 3,57 kg Wasser zugegeben. Danach wurde der Mischerinhalt bei 1500 Upm gerührt, dabei fand eine Temperaturerhöhung auf ca. 50°C statt. Nach 6 min Granulierzeit wurden nochmals 0,94 kg Wasser zugesetzt und nochmals 6 min weiter granuliert. Das erhaltene Granulat wurde im Vakuumtrockenschrank bei 80 °C und 50 mbar getrocknet.

## Tabelle 1: Siebanalyse, Rieselfähigkeit, Abriebfestigkeit und Schüttdichte der erhaltenen Granulate

| | | Beispiel 2.1 | Beispiel 2.2 | Beispiel 2.3 | Beispiel 2.4 |
|---|---|---|---|---|---|
| Siebanalyse [%] | < 63 μm | 1,3 | 4,4 | 1,4 | 8,6 |
| | 63 – 100 μm | 2,8 | 0,4 | 0,8 | 12,6 |
| | 100 – 200 μm | 7,8 | 10,8 | 0,6 | 30,1 |
| | 200 – 315 μm | 17,2 | 9,5 | 0,8 | 22,3 |
| | 315 – 500 μm | 47,5 | 14,5 | 2,3 | 24,7 |
| | 500 – 710 μm | 19,0 | 30,5 | 89,8 | 1,6 |
| | 710 – 850 μm | 3,3 | 21,5 | 3,6 | 0,0 |
| | > 850 μm | 1,2 | 8,4 | 0,7 | 0,0 |
| Rieselfähigkeit [Note] | | 2 | 2 | 3 | 3 |
| Anteil < 63 μm [%] | | 1,3 | 4,4 | 1,4 | 8,6 |
| Schüttdichte [g/L] | | 587 | 617 | 532 | 426 |
| Abrieb < 63 μm [%] | nach 3 min | 1,3 | 7,9 | 1,6 | 8,6 |
| | nach 15 min | 4,4 | 14,8 | 4,8 | 17,0 |
| | Differenz zwischen 3 und 15 min | 3,1 | 6,9 | 3,2 | 8,4 |

**Patentansprüche**

1. Abriebfeste und rieselfähige Glycocyamin-haltige Formlinge, mit einem Schüttgewicht zwischen 350 und 850 kg/m$^3$, einem Kornspektrum von 32 bis 2750 μm und einem Glycocyamingehalt von 55 bis 99,9 Gew.-%, bezogen auf das Gesamtgewicht.

2. Formlinge nach Anspruch 1, **dadurch gekennzeichnet, dass** diese organische oder anorganische Bindemittel, insbesondere Methylcellulose, Ethylcellulose, Carboxymethylcellulose, Carboxyethylcellulose, Carboxypropylcellulose, Hydroxypropylmethylcellulose, Hydroxymethylcellulose, mikrokristalline Cellulose, Ethylmethylcellulose und andere Cellulose-Derivate, Stärke, Hydroxypropylstärke, native Stärke, pregelatinisierte oder modifizierte Stärke, Zucker, Zuckersirup, Dextrin, Gelatine, Propylvinylalkohol, Polyvinylpyrrolidon, Xanthan, Glycin, Glycocyamin-Salze, Gummiarabicum, Natriumchlorid, Natriumcarbonat, Natriumhydrogencarbonat, Glycerin und gegebenenfalls Mischungen dieser Bindemittel, in Mengen von 0,05 bis 15 Gew.-%, bevorzugt 0,1 bis 1,5 Gew.-% enthalten.

3. Formlinge nach einem der Ansprüche 1 oder 2, **dadurch gekennzeichnet, dass** sie einem Kornspektrum von 32 μm bis 1000 μm besitzen, wobei vorzugsweise weniger als 10 Gew.-% der Partikel unter 100 μm und weniger als 10 Gew.-% der Partikel über 850 μm liegen, oder/und dass diese ein Schüttgewicht zwischen 400 und 800 kg/m$^3$, vorzugsweise 450 bis 750 kg/m$^3$ aufweisen.

4. Formlinge nach einem der Ansprüche 1 bis 3, **dadurch gekennzeichnet, dass** diese einen Glycocyamingehalt von 85 bis 99 Gew.-%, vorzugsweise 95 bis 98,5 Gew.-% aufweisen, oder/und dass diese bis zu 40 Gew.-% weitere ernährungsphysiologisch wirksame Stoffe der Reihe Kohlenhydrate, Fette, Aminosäuren, Proteine, Vitamine, Mineralstoffe, Spurenelemente sowie deren Derivate und Mischungen daraus enthalten oder/und dass diese als Fließhilfsmittel eine hydrophile oder hydrophobe Kieselsäure oder Zuschlagstoffe auf silikatischer Basis oder Fettsäuren oder deren Salze oder Mischungen dieser Fließhilfsmittel in Mengen von 0,01 bis 5 Gew.-% enthalten.

5. Formlinge nach einem der Ansprüche 1 bis 4, **dadurch gekennzeichnet, dass** sie eine Rieselfähigkeit der Note 3, vorzugsweise der Note 2 oder 1 besitzen, oder/und dass diese eine Abriebfestigkeit von unter 12 Gew.-%, vorzugsweise unter 10 Gew.-% und besonders bevorzugt unter 4 Gew.-% aufweisen.

6. Verfahren zur Herstellung abriebfester und rieselfähiger Glycocyaminhaltiger Formlinge nach einem der Ansprüche 1 bis 5, **dadurch gekennzeichnet, dass** diese durch Mischgranulation einer Zusammensetzung aus Glycocyamin und Wasser und anschließendes Trocknen der erhaltenen Granulate erhalten werden.

7. Verfahren nach Anspruch 6, **dadurch gekennzeichnet, dass** als Granulatoren Intensivmischer, Vertikal-Granulatoren, Sprühgranulatoren, Ringschichtgranulatoren und Pflugscharmischer verwendet werden.

8. Verfahren zur Herstellung abriebfester und rieselfähiger Glycocyaminhaltiger Formlinge nach einem der Ansprüchen 1 bis 5, **dadurch gekennzeichnet, dass** diese durch formgebende Extrudierung einer Zusammensetzung aus Glycocyamin und Wasser und anschließendes Trocknen der erhaltenen Extrudate erhalten werden.

9. Verfahren nach Anspruch 8, **dadurch gekennzeichnet, dass** als Extruder Einschneckenextruder, Zweischneckenextruder, Ringmatrizenpressen und Kollerwerke verwendet werden.

10. Verfahren nach einem der Ansprüche 6 bis 9, **dadurch gekennzeichnet, dass** man ein aus dem Herstellprozess abgetrenntes, wasserfeuchtes Glycocyamin mit einer Restfeuchte von 15 bis 25 Gew.-% verwendet.

11. Verfahren nach einem der Ansprüche 6 bis 10, **dadurch gekennzeichnet, dass** die eingesetzte Mischung zwischen 40 und 93 Gew.-% Glycocyamin, zwischen 7 und 60 Gew.-% Wasser, zwischen 0 und 15 Gew.-% Bindemittel und 0 bis 40 Gew.-% eines weiteren ernährungsphysiologisch wirksamen Stoffes enthält.

12. Verfahren nach einem der Ansprüche 6 bis 11, **dadurch gekennzeichnet, dass** das verwendete Glycocyamin einen mittleren Korndurchmesser von < 95 $\mu$m, vorzugsweise < 25 $\mu$m und besonders bevorzugt < 15 $\mu$m besitzt.

13. Verfahren nach einem der Ansprüche 6 bis 12, **dadurch gekennzeichnet, dass** das verwendete Glycocyamin zu mehr als 40 Gew.-% in amorpher Form vorliegt oder/und dass das verwendete Glycocyamin aus Glycin und Cyanamid in einem wässrigen Lösungsmittel unter Zugabe einer Base hergestellt wurde.

14. Verfahren nach einem der Ansprüche 6 bis 13, **dadurch gekennzeichnet, dass** die erhaltenen Glycocyaminhaltigen Formlinge, gegebenenfalls unter Vakuum, bei Temperaturen von 50 bis 130°C getrocknet werden.

15. Verwendung der abriebfesten und rieselfähigen Glycocyamin-haltigen Formlinge nach einem der Ansprüche 1 bis 5, **dadurch gekennzeichnet, dass** diese als Futtermitteladditiv eingesetzt werden.

**Claims**

1. Abrasion-resistant and free-flowing glycocyamine-containing moulded blanks having a bulk density of between 350 and 850 kg/m$^3$, a grain size spectrum of from 32 to 2750 $\mu$m and a glycocyamine content of from 55 to 99.9 wt.%, based on the total weight.

2. Moulded blanks according to claim 1, **characterised in that** said moulded blanks contain organic or inorganic binders, in particular methylcellulose, ethylcellulose, carboxymethylcellulose, carboxyethylcellulose, carboxypropylcellulose, hydroxypropylmethylcellulose, hydroxymethylcellulose, microcrystalline cellulose, ethylmethylcellulose and other cellulose derivatives, starch, hydroxypropyl starch, native starch, pregelatinised or modified starch, sugar, sugar syrup, dextrin, gelatine, propyl vinyl alcohol, polyvinyl pyrrolidone, xanthan, glycine, glycocyamine salts, gum arabic, sodium chloride, sodium carbonate, sodium hydrogen carbonate, glycerol and optionally mixtures thereof in amounts of from 0.05 to 15 wt.%, preferably from 0.1 to 1.5 wt.%.

3. Moulded blanks according to either claim 1 or claim 2, **characterised in that** said moulded blank has a grain size spectrum of from 32 $\mu$m to 1000 $\mu$m, preferably less than 10 wt.% of the particles being below 100 $\mu$m, and less than 10 wt.% of the particles being above 850 $\mu$m, and/or **in that** said moulded blank has a bulk density of between 400 and 800 kg/m$^3$, preferably of from 450 to 750 kg/m$^3$.

4. Moulded blanks according to any of claims 1 to 3, **characterised in that** said moulded blanks have a glycocyamine content of from 85 to 99 wt.%, preferably of from 95 to 98.5 wt.%, and/or **in that** said moulded blanks contain up to 40 wt.% of other nutritionally active substances selected from the group containing carbohydrates, fats, amino acids, proteins, vitamins, minerals, trace elements, and derivatives and mixtures thereof, and/or **in that** said moulded blanks contain hydrophilic or hydrophobic silicic acid, or silicate-based additives, or fatty acids, or salts thereof or mixtures of these anti-caking agents in amounts of from 0.01 to 5 wt.%.

5. Moulded blanks according to any of claims 1 to 4, **characterised in that** said moulded blanks have a flowability of grade 3, preferably of grade 2 or 1, and/or **in that** said moulded blanks have an abrasion resistance of below 12 wt.%, preferably below 10 wt.%, and most preferably below 4 wt.%.

6. Method for producing abrasion-resistant and free-flowing glycocyamine-containing moulded blanks according to any of claims 1 to 5, **characterised in that** said moulded blanks are obtained by mixed granulation of a composition of glycocyamine and water and subsequent drying of the resulting granules.

7. Method according to claim 6, **characterised in that** vertical granulators, spray granulators, ring-layer granulators, or ploughshare mixers are used as granulator intensive mixers.

8. Method for producing abrasion-resistant and free-flowing glycocyamine-containing moulded blanks according to any of claims 1 to 5, **characterised in that** said moulded blanks are obtained by shaping-extrusion of a composition of glycocyamine and water and subsequent drying of the resulting extrudates.

9. Method according to claim 8, **characterised in that** single-screw extruders, twin-screw extruders, ring-die presses, and grinders are used as extruders.

10. Method according to any of claims 6 to 9, **characterised in that** a water-moist glycocyamine, separated off from the production process and having a residual moisture of 15 to 25 wt.%, is used.

11. Method according to any of claims 6 to 10, **characterised in that** the mixture used contains between 40 and 93 wt.% glycocyamine, between 7 and 60 wt.% water, between 0 and 15 wt.% binder, and from 0 to 40 wt.% of another nutritionally active substance.

12. Method according to any of claims 6 to 11, **characterised in that** the glycocyamine used has an average grain size diameter of < 95 μm, preferably < 25 μm, and most preferably < 15 μm.

13. Method according to any of claims 6 to 12, **characterised in that** more than 40 wt.% of the glycocyamine used is present in an amorphous form and/or **in that** the glycocyamine used is produced from glycine and cyanamide in an aqueous solvent with the addition of a base.

14. Method according to any of claims 6 to 13, **characterised in that** the obtained glycocyamine-containing moulded blanks are dried at temperatures of from 50 to 130 °C, optionally under vacuum.

15. Use of the abrasion-resistant and free-flowing glycocyamine-containing moulded blanks according to any of claims 1 to 5, **characterised in that** said moulded blanks are used as feed additives.

**Revendications**

1. Produits moulés contenant de la glycocyamine, résistants à l'abrasion et aptes à l'écoulement, avec une masse volumique apparente comprise entre 350 et 850 kg/m$^3$, un spectre granulométrique de 32 à 2750 μm et une teneur en glycocyamine comprise de 55 à 99,9 % en poids, par rapport au poids total.

2. Produits moulés selon la revendication 1, **caractérisés en ce qu'**ils contiennent des liants organiques ou inorganiques, en particulier de la méthylcellulose, de l'éthylcellulose, de la carboxyméthylcellulose, de la carboxyéthylcellulose, de la carboxypropylcellulose, de l'hydroxypropylméthylcellulose, de l'hydroxyméthylcellulose, de la cellulose microcristalline, de l'éthylméthylcellulose et d'autres dérivés de la cellulose, de l'amidon, de l'hydroxypropyl amidon, de l'amidon natif, de l'amidon prégélatinisé ou modifié, du sucre, du sirop de sucre, de la dextrine, de la gélatine, de l'alcool propylvinylique, de la polyvinylpyrrolidone, du xanthane, de la glycine, des sels de glycocyamine, de la gomme arabique, du chlorure de sodium, du carbonate de sodium, du bicarbonate de sodium, de la glycérine et le cas échéant des mélanges de ces liants, dans des quantités comprises de 0,05 à 15 % en poids, de préférence de 0,1 à 1,5 % en poids.

3. Produits moulés selon l'une des revendications 1 ou 2, **caractérisés en ce qu'**ils possèdent un spectre granulo-métrique de 32 $\mu$m à 1000 $\mu$m, avec de préférence moins de 10 % en poids des particules étant sous 100 $\mu$m et moins de 10 % en poids des particules étant au-dessus de 850 $\mu$m, et/ou **caractérisés en ce que** ceux-ci présentent une masse volumique apparente comprise entre 400 et 800 kg/m$^3$, de préférence de 450 à 750 kg/m$^3$.

4. Produits moulés selon l'une des revendications 1 à 3, **caractérisés en ce qu'**ils présentent une teneur en glycocyamine comprise de 85 à 99 % en poids, de préférence comprise de 95 à 98,5 % en poids, et/ou **caractérisés en ce qu'**ils contiennent jusqu'à 40 % en poids d'autres substances actives sur le plan physiologique et nutritionnel de la famille des hydrates de carbone, des graisses, des acides aminés, des protéines, des vitamines, des substances minérales, des oligo-éléments, ainsi que leurs dérivés et leurs mélanges et/ou **caractérisés en ce qu'**ils contiennent, en tant qu'agents anti-mottants, un acide silicique hydrophile ou hydrophobe ou des additifs à base de silicate ou des acides gras ou leurs sels, ou des mélanges de ces agents anti-mottants dans des quantités comprises entre 0,01 et 5 % en poids.

5. Produits moulés selon l'une des revendications 1 à 4, **caractérisés en ce qu'**ils possèdent une aptitude à l'écoulement de classe 3, de préférence de classe 2 ou 1, et/ou **caractérisés en ce que** ceux-ci présentent une résistance à l'abrasion inférieure à 12 % en poids, de préférence inférieure à 10 % en poids et de préférence encore inférieure à 4 % en poids.

6. Procédé de fabrication de produits moulés contenant de la glycocyamine, résistants à l'abrasion et aptes à l'écoulement, selon l'une des revendications 1 à 5, **caractérisé en ce que** ceux-ci sont obtenus par granulation d'une composition de glycocyamine et d'eau, puis par séchage des granulats obtenus.

7. Procédé selon la revendication 6, **caractérisé en ce que** des mélangeurs intensifs, des granulateurs verticaux, des granulateurs à pulvérisation, des granulateurs à couche annulaire (« RingSchichtgranulatoren ») et des mélangeurs à « socs de charrue » sont utilisés comme granulateurs.

8. Procédé de fabrication de produits moulés contenant de la glycocyamine, résistants à l'abrasion et aptes à l'écoulement, selon l'une des revendications 1 à 5, **caractérisé en ce que** ceux-ci sont obtenus par moulage par extrusion d'une composition de glycocyamine et d'eau, puis par séchage des extrudats obtenus.

9. Procédé selon la revendication 8, **caractérisé en ce que** des extrudeuses monovis, des extrudeuses bivis, des presses à matrice annulaire et des broyeurs à meule (« Kollerwerke ») sont utilisés comme extrudeuses.

10. Procédé selon l'une des revendications 6 à 9, **caractérisé en ce que** de la glycocyamine humide, séparée du processus de fabrication est utilisée, avec une humidité résiduelle comprise entre 15 et 25 % en poids.

11. Procédé selon l'une des revendications 6 à 10, **caractérisé en ce que** le mélange utilisé comprend entre 40 et 93 % en poids de glycocyamine, entre 7 et 60 % en poids d'eau, entre 0 et 15 % en poids de liants et entre 0 et 40 % en poids d'une autre substance active sur le plan physiologique et nutritionnel.

12. Procédé selon l'une des revendications 6 à 11, **caractérisé en ce que** la glycocyamine utilisée possède un diamètre moyen de grains de < 95 $\mu$m, de préférence de < 25 $\mu$m et de préférence encore < 15 $\mu$m.

13. Procédé selon l'une des revendications 6 à 12, **caractérisé en ce que** la glycocyamine utilisée se présente à plus de 40 % en poids sous une forme amorphe et/ou **caractérisé en ce que** la glycocyamine utilisée a été fabriquée à partir de glycine et de cyanamide dans un solvant aqueux avec addition d'une base.

14. Procédé selon l'une des revendications 6 à 13, **caractérisé en ce que** les produits moulés contenant de la glycocyamine sont séchés à des températures comprises de 50 à 130°C, éventuellement sous vide.

15. Utilisation de produits moulés contenant de la glycocyamine, résistants à l'abrasion et aptes à l'écoulement selon l'une des revendications 1 à 5, **caractérisée en ce qu'**ils sont utilisés comme additifs alimentaires pour animaux.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1051914 A
- EP 1758463 A
- US 2654779 A, Vassel, B.
- US 2620354 A, Vassel, B.
- US 1967400 A, Fischl, S.

**In der Beschreibung aufgeführte Nicht-Patentliteratur**

- **FROMM, E.** *Justus Liebigs Ann. Chem.,* 1925, vol. 442, 130-149
- **WHEELER, H.I. ; MERRIAM, H.F.** *Am. Chem. Journal,* 1903, vol. 29, 478-492
- *Feed Tech,* 10. September 2005, 23-26